# EUROPÄISCHE PATENTSCHRIFT

(11) **EP 2 953 534 B1**
(45) Veröffentlichungstag und Bekanntmachung des Hinweises auf die Patenterteilung: **16.10.2019**
(21) Anmeldenummer: 14703265.0
(22) Anmeldetag: 04.02.2014
(51) Int. Cl.: A61B 5/055, A61B 5/00, A61B 6/04

(54) **ANORDNUNG ZUM VORBEREITEN UND POSITIONIEREN VON MEDIZINISCH ZU BEHANDELNDEN UND/ODER ZU VERSORGENDEN PATIENTEN**
ARRANGEMENT FOR PREPARING AND POSITIONING PATIENTS TO BE TREATED AND/OR CARED FOR MEDICALLY
INSTALLATION DE PRÉPARATION ET DE POSITIONNEMENT DE PATIENTS À TRAITER ET/OU À PRENDRE EN CHARGE MÉDICALEMENT

(30) Priorität: 06.02.2013 DE 102013002183
(43) Veröffentlichungstag der Anmeldung: 16.12.2015
(73) Patentinhaber: gKteso GmbH, 86399 Bobingen (DE)
(72) Erfinder: KÜBLER, Guido, 86399 Bobingen (DE)
(74) Vertreter: Grape & Schwarzensteiner
(86) Internationale Anmeldenummer: PCT/EP2014/000292
(87) Internationale Veröffentlichungsnummer: WO 2014/121919

(56) Entgegenhaltungen:
- US-A- 5 259 011
- US-A- 6 094 760
- US-A1- 2004 001 571

## Beschreibung

Die vorliegende Erfindung betrifft eine Anordnung bzw. Vorrichtung zum Vorbereiten und Positionieren von medizinisch zu behandelnden und/oder zu versorgenden Patienten.

Derartige Anordnungen sind allgemein bekannt. Beispielsweise sind aus den US 6,615,428 B1, US 6,955,464 B1 oder US 7,216,383 B2 Vorrichtungen zum Vorbereiten und Positionieren von Patienten bekannt, die einer diagnostischen Untersuchung mittels Röntgenstrahlung, Computertomographie (CT), Kernspinresonanztomographie (MRI) oder Positronenemissionstomographie (PET) unterzogen werden. So bestehen diese Vorrichtungen jeweils aus einer Patientenliege, die dem entsprechenden Diagnostikgerät unmittelbar zu- bzw. vorgeordnet ist. Die Patientenliege ist dabei direkt in das bzw. aus dem Diagnostikgerät ein- bzw. ausfahrbar ausgebildet. Alternativ dazu kann die Patientenliege hin zu bzw. weg von einer stirnseitig ausgerichteten Unterlage des Diagnostikgerätes ein- bzw. ausfahrbar ausgebildet sein, was jedoch eine zusätzliche Umlagerung des Patienten erforderlich macht. Besonders nachteilig bei allen diesen Vorrichtungen ist die unmittelbare Zu- bzw. Vorordnung der Patientenliege zu bzw. gegenüber dem Diagnostikgerät. Die Patientenliege und das Diagnostikgerät sind daher in demselben Behandlungsraum angeordnet. Das Vorbereiten und Positionieren des medizinisch zu behandelnden und/oder zu versorgenden Patienten und die medizinische Behandlung und/oder Versorgung erfolgen somit in nächster Nähe und in zeitlicher Abfolge voneinander. Ein Vorbereiten und Positionieren eines medizinisch zu behandelnden und/oder zu versorgenden Patienten und eine medizinische Behandlung und/oder Versorgung eines anderen Patienten können niemals gleichzeitig bzw. parallel zueinander erfolgen. Dies Alles führt zu ausgesprochen langen Vorbereitungs- und somit Behandlungsintervallen des Patienten und damit zu einem hohen Zeit- und Kostenaufwand in der medizinischen Behandlung und/oder Versorgung des/der Patienten insgesamt.

Darüber hinaus sind in den US 2009/0070936 A1, US 2012/0158017 A1, US 2012/0174317 A1 und US 6,094,760 A computergestützte Robotersysteme zum Positionieren von Patienten gegenüber einer Vorrichtung zur medizinischen Behandlung und/oder Versorgung des Patienten beschrieben. Diese Robotersysteme weisen sämtlich eine Reihe erheblicher Nachteile auf. Demnach besitzen diese Robotersysteme ausnahmslos eine sehr aufwendige Bauweise und sind damit einhergehend besonders wartungsintensiv. Weiterhin sind diese Robotersysteme aufgrund ihrer Bewegungsfreiheit und vielen Freiheitsgrade ausgesprochen schwer und allenfalls durch sehr erfahrenes Fachpersonal zu betätigen und zu handhaben. Infolge von Bewegungsfreiheit und vielen Freiheitsgraden sind gleichzeitig hohe Kollisionsrisiken mit der Vorrichtung zur medizinischen Behandlung und/oder Versorgung des Patienten vorhanden, was zugleich ein erhebliches Verletzungspotential für Patienten, Bedienungspersonal und Ärzten darstellt. Von größtem Nachteil aber bei diesen Robotersystemen hat sich in der Praxis die Tatsache herausgestellt, dass diese Robotersysteme grundsätzlich nur einer einzigen Vorrichtung zur medizinischen Behandlung und/oder Versorgung des Patienten zugeordnet, mithin unmittelbar in dem eigentlichen Behandlungsraum angeordnet sind. Das Vorbereiten und Positionieren des medizinisch zu behandelnden und/oder zu versorgenden Patienten und die medizinische Behandlung und/oder Versorgung erfolgen insoweit in unmittelbarer räumlicher Nähe und zeitlicher Abhängigkeit. Ein Vorbereiten und Positionieren eines medizinisch zu behandelnden und/oder zu versorgenden Patienten und eine medizinische Behandlung und/oder Versorgung eines anderen Patienten können demzufolge niemals gleichzeitig stattfinden. Dies Alles führt einerseits zu extrem hohen Herstellungs-, Betriebs- und Wartungs- sowie Reparaturkosten dieser Robotersysteme insgesamt wie ebenso andererseits zu ausgesprochen langen Vorbereitungs- und somit Behandlungsintervallen des Patienten.

Weiterhin sind in den US 3,504,386 A, US 5,475,884 A, US 6,094,760 A und US 2004/0001571 A1 oder der DE 101 27 210 A1 kombinierte Patientenlagerungs- und Transportsysteme bekannt, die eine Patientenliege und einen Transportwagen zum Transport der Patientenliege mit Patient umfassen. Diese Patientenlagerungs- und Transportsysteme haben sich in der Praxis jedoch allesamt als ausgesprochen nachteilig erwiesen. So ist ein automatischer oder auch ein nur weitgehend automatischer Transport von einem Vorbereitungsraum zu einem Behandlungsraum, und umgekehrt, oder zu einem weiteren Behandlungsraum nicht möglich. Weiterhin ist ein einfacher Transportvorgang zwischen Vorbereitungsraum und Behandlungsraum ausgeschlossen. Transportwagen sind aufgrund von deren Abmessungs- und Gewichtsverhältnissen einschließlich dem zu transportierenden Patienten in aller Regel nur äußerst schwer handhabbar. Dies um so mehr, wenn zwischen Vorbereitungsraum und Behandlungsraum Rampen, abgewinkelte Gänge oder dergleichen zu überwinden sind. Es bedarf vielmehr zusätzlichen Personals, d.h. wenigstens einer zusätzlichen Person, was wiederum einen erhöhten Zeit- und Kostenaufwand für den eigentlichen Transportvorgang nach sich zieht. Nicht zuletzt hieraus resultierend ist bei dem Transportvorgang das Risiko einer Kollision auf dem Weg zwischen Vorbereitungsraum und Behandlungsraum enorm groß, selbst wenn zwei Personen zum Handhaben des Transportwagens vorgesehen sind. Zudem lässt sich der Transportvorgang nicht von örtlich anzutreffenden Bodengegebenheiten und damit ohne jegliche Erschütterungen vornehmen. Solche Kollisionen und/oder Erschütterungen wiederum wirken sich auf den vorbereiteten und positionierten Patienten gänzlich nachteilig aus, insoweit, als die in dem Vorbereitungsraum vorgenommene Vorbereitung und exakt eingestellte und definierte Positionierung des Patienten erheblich beeinträchtigt oder sogar aufgehoben wird und infolgedessen nach dem Transportvorgang in dem Behandlungsraum erneut durchgeführt werden muss. Schließlich sind bei diesen Patientenlagerungs- und Transportsystemen erhebliche Probleme mit der erforderlich werdenden Umlagerung des Patienten von der Patientenliege auf den Tisch zu dessen medizinischen Behandlung und/oder Versorgung verbunden. Auch durch eine solche Umlagerung wird die in dem Vorbereitungsraum vorgenommene Vorbereitung und exakt eingestellte und definierte Positionierung des Patienten erheblich beeinträchtigt oder sogar aufgehoben, so dass in dem Behandlungsraum eine erneute Vorbereitung und Positionierung des Patienten, zumindest eine zusätzliche Überprüfung dessen notwendig wird.

Schließlich ist aus der US 5,259,011 A eine Anordnung zum Vorbereiten und Positionieren von medizinisch zu behandelnden und/oder zu versorgenden Patienten umfassend wenigstens zwei Vorbereitungsbereiche mit wenigstens einer in dem jeweiligen Vorbereitungsbereich angeordneten Vorrichtung zum Vorbereiten und Positionieren eines Patienten und mindestens einer in einem Behandlungsbereich angeordneten Vorrichtung zur medizinischen Behandlung und/oder Versorgung des Patienten. Dabei soll ein Patientenbett mit einem Schienen-basierten oder Förderband-System transportiert werden.

Der vorliegenden Erfindung liegt daher die Aufgabe zugrunde, eine Anordnung bzw. Vorrichtung zum Vorbereiten und Positionieren von medizinisch zu behandelnden und/oder zu versorgenden Patienten zur Verfügung zu stellen, mit welcher sich die obigen Nachteile verhindern lassen, welche mithin eine zeitgleiche bzw. parallele Vorbereitung und Positionierung und anschließende Behandlung und/oder Versorgung von Patienten ermöglicht, zugleich konstruktiv besonders einfach, kompakt und zuverlässig ist, weitgehend automatisch, zudem einfach, schnell und für Patienten, Bedienungspersonal und Ärzten gefahrlos handhabbar ist sowie zu einer erheblichen Kostenreduzierung bei der Behandlung und/oder Versorgung von Patienten führt.

Diese Aufgabe wird auf überraschend einfache Weise durch die Merkmale des Anspruchs 1 gelöst.

Durch die Ausgestaltung der erfindungsgemäßen Anordnung zum Vorbereiten und Positionieren von medizinisch zu behandelnden und/oder zu versorgenden Patienten gemäß Anspruch 1 ist eine zeitgleiche bzw. parallele Vorbereitung und Positionierung und anschließende Behandlung und/oder Versorgung von Patienten ermöglicht. Damit einhergehend lassen sich Auslastung der medizinischen Räumlichkeiten und/oder Vorrichtungen und Geräte zur Behandlung und/oder Versorgung des Patienten wesentlich effizienter gestalten sowie folglich die Zahl von medizinisch zu behandelnden und/oder zu versorgenden Patienten pro Zeiteinheit erheblich steigern. Zugleich wird eine konstruktiv besonders einfache, kompakte und zuverlässige Bauweise der erfindungsgemäßen Anordnung erreicht. Nicht zuletzt hieraus resultierend gestaltet sich die Bauweise der erfindungsgemäßen Anordnung wartungsarm. Weiterhin ist ein nahezu automatischer Transport des Patienten aus dem Vorbereitungsbereich in einen Behandlungsbereich und anschließend zurück in den Vorbereitungsbereich oder einen anderen Behandlungsbereich möglich, der sich zudem ausgesprochen einfach und schnell handhaben lässt, und zwar ohne Einsatz von Personal. Jegliches Kollisions- und/oder Verletzungsrisiko für Patienten, Bedienungspersonal und Ärzten ist aufgrund der linearen Bewegungsmimik und Automatisierung ausgeschlossen. Der Transportvorgang selbst ist daher keinen Kollisionen unterworfen. Hinzu kommt, dass der Transportvorgang vorzugsweise nicht bodengeführt erfolgt und somit von möglicherweise durch Bodenunebenheiten hervorgerufenen Erschütterungen nicht beeinträchtigt ist. Eine in dem Vorbereitungsbereich vorgenommene Vorbereitung und exakt eingestellte und definierte Positionierung des Patienten auf der Unterlage bleibt unverändert erhalten. Schließlich ist bei der erfindungsgemäßen Anordnung auch keine Umlagerung des Patienten notwendig. Beides macht eine erneute Vorbereitung und Positionierung des Patienten, zumindest eine zusätzliche Überprüfung dessen überflüssig. Mit der medizinischen Behandlung und/oder Versorgung des Patienten kann vielmehr unverzüglich nach dem Transport im Behandlungsbereich begonnen werden. Im Ergebnis lassen sich mit der erfindungsgemäßen Anordnung eine erhebliche Reduzierung von Herstellungs-, Betriebs- und Wartungs- sowie Reparaturkosten und von Zeit-, Personal- und damit Kostenaufwand bei der Behandlung und/oder Versorgung von Patienten insgesamt erreichen.

Weitere vorteilhafte Einzelheiten der erfindungsgemäßen Anordnung sind in den Ansprüchen 2 bis 14 beschrieben.

Von besonderem Vorteil sind die Merkmale des Anspruchs 2, wonach die wenigstens eine Vorbereitungs- und Positionierungsvorrichtung und die mindestens eine Behandlungs- und/oder Versorgungsvorrichtung zueinander linear angeordnet, um mithin auf einer Geraden aufeinander zu und voneinander weg bewegt werden zu können.

Vorzugsweise liegt es im Rahmen der Erfindung, dass die wenigstens eine Vorbereitungs- und Positionierungsvorrichtung nach Anspruch 3 zwischen zwei oder mehreren Behandlungs- und/oder Versorgungsvorrichtungen angeordnet ist.

In diesem Zusammenhang können zwei oder mehrere Vorbereitungs- und Positionierungsvorrichtungen nach Anspruch 4 in ganz vorteilhafter Weise parallel zueinander angeordnet sein.

In alternativer oder kumulativer Weise dazu können zwei oder mehrere Vorbereitungs- und Positionierungsvorrichtungen jedoch nach Anspruch 5 in ebenso vorteilhafter Weise im Wesentlichen auf einem Teilkreis oder in einem Kreis nebeneinander um die mindestens eine Behandlungs- und/oder Versorgungsvorrichtung angeordnet werden.

Weiterhin ist erfindungsgemäß vorgesehen, dass zwei oder mehrere Behandlungs- und/oder Versorgungsvorrichtungen nach Anspruch 6 parallel nebeneinander und/oder hintereinander und/oder versetzt zueinander angeordnet sind.

Besonders zweckmäßig sind die konstruktiven Maßnahmen nach Anspruch 7. Demnach ist die wenigstens eine Vorbereitungs- und Positionierungsvorrichtung derart ausgebildet, dass die von der linear geführten Teleskopeinrichtung abgestützte formstabile Unterlage der wenigstens einen Vorbereitungs- und Positionierungsvorrichtung vollautomatisch aus dem Vorbereitungsbereich in den Behandlungsbereich, und umgekehrt, verbringbar ist.

Von besonderem Vorteil sind die Merkmale des Anspruchs 8, dass nämlich die linear geführte Teleskopeinrichtung der wenigstens einen Vorbereitungs- und Positionierungsvorrichtung elektromotorisch und/oder pneumatisch und/oder hydraulisch antreibbar ist. Ein elektromotorischer Antrieb ist grundsätzlich bevorzugt. Von Vorteil kann es aber sein, alternativ oder kumulativ einen auf pneumatischem und/oder hydraulischem Wirkprinzip basierenden Antrieb vorzusehen, wenn dies etwa durch zusätzliche Gegebenheiten oder Randbedingungen erforderlich ist. Beispielsweise kann ein pneumatischer oder hydraulischer Antrieb von Vorteil sein, wenn als Vorrichtung zur medizinischen Behandlung und/oder Versorgung des Patienten ein Diagnostikgerät mittels Röntgenstrahlung, Computertomographie (CT), Kernspinresonanztomographie (MRI) oder Positronenemissionstomographie (PET) zum Einsatz kommt.

Nach Anspruch 9 ist in vorteilhafter Weise erfindungsgemäß vorgesehen, dass die linear geführte Teleskopeinrichtung der wenigstens einen Vorbereitungs- und Positionierungsvorrichtung vollständig oder teilweise auf einer Basis oder einem Fundament abstützbar und führbar ist.

Zweckmäßigerweise sind die formstabile Unterlage und die linear geführte Teleskopeinrichtung der wenigstens einen Vorbereitungs- und Positionierungsvorrichtung nach Anspruch 10 miteinander unlösbar verbunden.

In alternativer Ausgestaltung der Erfindung ist es möglich, dass die formstabile Unterlage und die linear geführte Teleskopeinrichtung der wenigstens einen Vorbereitungs- und Positionierungsvorrichtung nach Anspruch 11 auch miteinander lösbar verbunden sind und die formstabile Unterlage von der linear geführten Teleskopeinrichtung auf einen Unter- oder Gestelltisch der mindestens einen Behandlungs- und/oder Versorgungsvorrichtung absetzbar und von dem Unter- oder Gestelltisch lösbar aufnehmbar und abstützbar ist.

Vorzugsweise sind die formstabile Unterlage und die linear geführte Teleskopeinrichtung der wenigstens einen Vorbereitungs- und Positionierungsvorrichtung entsprechend den Merkmalen des Anspruchs 12 zueinander relativ verdrehbar ausgebildet.

Alternativ oder kumulativ dazu können die erfindungsgemäßen Merkmale des Anspruchs 13 von Vorteil sein, wonach die formstabile Unterlage der wenigstens einen Vorbereitungs- und Positionierungsvorrichtung und der Unter- oder Gestelltisch der mindestens einen Behandlungs- und/oder Versorgungsvorrichtung zueinander relativ verdrehbar ausgebildet sind und/oder der Unter- oder Gestelltisch der mindestens eine Behandlungs- und/oder Versorgungsvorrichtung und ein/e den Unter- oder Gestelltisch abstützende/s Basis oder Fundament der mindestens einen Behandlungs- und/oder Versorgungsvorrichtung relativ verdrehbar ausgebildet sind.

Schließlich liegt es noch im Rahmen der Erfindung, nach Anspruch 14 die Vorbereitungs- und Positionierungsvorrichtungen zueinander identisch auszubilden und/oder die Behandlungs- und/oder Versorgungsvorrichtungen zueinander identisch oder unterschiedlich auszubilden.

Weitere Merkmale, Vorteile und Einzelheiten der Erfindung ergeben sich aus der nachfolgenden Beschreibung einer bevorzugten Ausführungsform der Erfindung sowie anhand der Zeichnungen. Hierbei zeigen:
- Fig. 1A und 1B: eine schematische Seitenansicht und eine Draufsicht auf eine beispielhafte Ausführungsform einer Anordnung zum Vorbereiten und Positionieren von medizinisch zu behandelnden und/oder zu versorgenden Patienten in einer Anfangsstellung,
- Fig. 1C und 1D: eine schematische Seitenansicht und eine Draufsicht auf die beispielhafte Ausführungsform der Anordnung nach den Fig. 1A und 1B in einer Zwischenstellung,
- Fig. 1E und 1F: eine schematische Seitenansicht und eine Draufsicht auf die beispielhafte Ausführungsform der Anordnung nach den Fig. 1A und 1B in einer Endstellung oder in einer anderen Zwischenstellung,
- Fig. 1G und 1H: eine schematische Seitenansicht und eine Draufsicht auf die beispielhafte Ausführungsform der Anordnung nach den Fig. 1A und 1B in einer weiteren Endstellung,
- Fig. 2: eine schematische Draufsicht auf eine weitere beispielhafte Ausführungsform einer ausgebildeten Anordnung in einer Zwischenstellung, in vergrößerter Darstellung,
- Fig. 3A und 3B: schematische Draufsichten auf eine Ausführungsform der erfindungsgemäßen Anordnung in einer Anfangsstellung und einer Zwischenstellung,
- Fig. 4: eine schematische Draufsicht auf eine andere Ausführungsform der erfindungsgemäßen Anordnung,
- Fig. 5: eine schematische Draufsicht auf eine wiederum andere Ausführungsform der erfindungsgemäßen Anordnung,
- Fig. 6: eine schematische Draufsicht auf eine noch andere Ausführungsform der erfindungsgemäßen Anordnung,
- Fig. 7: eine schematische Draufsicht auf eine wiederum abgewandelte Ausführungsform der erfindungsgemäßen Anordnung, und
- Fig. 8A, 8B und 8C: schematische Draufsichten auf eine weitere Ausführungsform der erfindungsgemäßen Anordnung in verschiedenen Stellungen, nämlich in einer Anfangs-, Zwischen und Endstellung.

Bei der nachfolgenden Beschreibung von verschiedenen Ausführungsformen einer erfindungsgemäßen Anordnung 10 sind einander entsprechende, gleiche Bauteile jeweils mit identischen Bezugsziffern versehen.

Die erfindungsgemäße Anordnung 10 ist zum Vorbereiten und Positionieren von medizinisch zu behandelnden und/oder zu versorgenden Patienten 12 vorgesehen.

Eine beispielhafte Anordnung 10 umfasst wenigstens eine Vorrichtung 14 zum Vorbereiten und Positionieren des Patienten 12 in einem Vorbereitungsbereich 16 oder dergleichen Vorbereitungsraum. Der Vorbereitungsbereich 16 kann gleichermaßen als Ruheraum oder dergleichen im Anschluss an eine medizinischen Behandlung und/oder Versorgung des Patienten 12 vorgesehen sein bzw. zum Einsatz kommen.

Weiterhin umfasst die Anordnung 10 nach der Erfindung mindestens eine Vorrichtung 18 zur medizinischen Behandlung und/oder Versorgung des Patienten 12 in einem Behandlungsbereich 20 oder dergleichen Behandlungsraum. Die medizinische Behandlungs- und/oder Versorgungsvorrichtung 18 kann beispielsweise als ein Diagnostikgerät zur diagnostischen Untersuchung mittels Röntgenstrahlung, Computertomographie (CT), Kernspinresonanztomographie (MRI) oder Positronenemissionstomographie (PET) ausgebildet sein. Ebenso ist es denkbar, die medizinische Behandlungs- und/oder Versorgungsvorrichtung 18 zum Beispiel als Gantry oder dergleichen zur Bestrahlung des Patienten 12 oder als Operationsraum bzw. -saal zum operativen oder chirurgischen Eingriff bzw. zur Operation des Patienten 12 vorzusehen. Auch jede andere Kombination davon untereinander oder andere Behandlungs- und/oder Versorgungsvorrichtungen 18 sind bei der erfindungsgemäßen Anordnung 10 möglich.

Der Vorbereitungsbereich 16 und der Behandlungsbereich 20 sind bei der erfindungsgemäßen Anordnung 10 insoweit voneinander in ihrer Funktion unterschiedlich. Weiterhin sind der Vorbereitungsbereich 16 und der Behandlungsbereich 20 bei der erfindungsgemäßen Anordnung 10 voneinander räumlich getrennt bzw. beabstandet. Der Vorbereitungsbereich 16 und der Behandlungsbereich 20 können dabei zu einem gemeinsamen Raum oder Gebäudeteil zusammengefasst bzw. in einem gemeinsamen Raum oder Gebäudeteil untergebracht sein. Es muss allerdings gewährleistet sein, dass der Vorbereitungsbereich 16 und der Behandlungsbereich 20 weitgehend abgeschlossen sind bzw. durch geeignete Maßnahmen, etwa durch Sichtschutzmaßnahmen, wie mobile Wände oder Trennvorhänge etc., voneinander abgetrennt sind, um etwa eine gegenseitige Einsichtmöglichkeit durch andere Patienten zu verhindern. Vorzugsweise sind der Vorbereitungsbereich 16 und der Behandlungsbereich 20 jedoch als gesonderte Räume oder Gebäudeteile ausgebildet und voneinander durch feste Wände, Gebäudemauern etc. abgeteilt. Der Vorbereitungsbereich 16 und der Behandlungsbereich 20 können zueinander unmittelbar benachbart, d.h. beispielsweise nebeneinander liegen, oder aber ebenso durch weitere dazwischen angeordnete Vorbereitungs- und/oder Behandlungsbereiche 16, 20 voneinander beabstandet sein.

Die wenigstens eine Vorbereitungs- und Positionierungsvorrichtung 14 umfasst eine formstabile Unterlage 22 für die Aufnahme, Vorbereitung und Positionierung des Patienten 12 in dem Vorbereitungsbereich 16. Der Patient 12 wird von der formstabilen Unterlage 22 aufgenommen bzw. auf diese (auf-)gelegt und von dieser abgestützt und getragen. Vor der medizinischen Behandlung und/oder Versorgung wird der Patient 12 dabei in geeigneter Weise vorbereitet und entsprechend der vorzusehenden Behandlungs- und/oder Versorgungsmaßnahmen positioniert sowie ausgerichtet. Durch die Formstabilität der Unterlage 22 ist sichergestellt, dass die einmal eingenommene Lage bzw. Position des Patienten 12, sofern sich dieser nicht selbst bewegt, beibehalten werden kann und somit von Außen nicht weiter veränderbar ist.

Darüber hinaus weist die wenigstens eine Vorbereitungs- und Positionierungsvorrichtung 14 eine linear geführte Teleskopeinrichtung 24 auf, welche wiederum die formstabile Unterlage 22 abstützt. Mittels der linear geführten Teleskopeinrichtung 24 kann der Patient 12, der in dem Vorbereitungsbereich 16 auf der formstabilen Unterlage 22 vorbereitet und positioniert wurde und von dieser in dieser Lage unverändert abgestützt ist, aus dem Vorbereitungsbereich 16 zu der mindestens einen Behandlungs- und/oder Versorgungsvorrichtung 18 in dem Behandlungsbereich 20, und umgekehrt, verbracht bzw. transportiert werden.

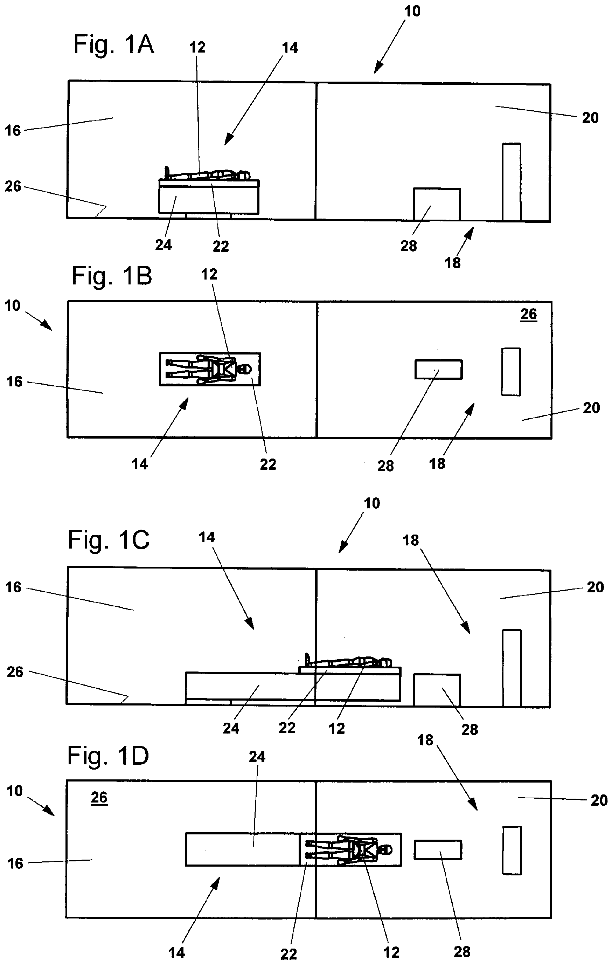

Die Fig. 1A bis 1H zeigen eine erste beispielhafte Ausführungsform der Anordnung 10, die eine Vorbereitungs- und Positionierungsvorrichtung 14 und eine medizinische Behandlungs- und/oder Versorgungsvorrichtung 18 umfasst. Die eine Vorbereitungs- und Positionierungsvorrichtung 14 und die eine medizinische Behandlungs- und/oder Versorgungsvorrichtung 18 sind jeweils in einem gesonderten Vorbereitungsbereich 16 oder Vorbereitungsraum und Behandlungsbereich 20 oder Behandlungsraum untergebracht.

Die Vorbereitungs- und Positionierungsvorrichtung 14 und die Behandlungs- und/oder Versorgungsvorrichtung 18 sind zueinander linear angeordnet, befinden sich somit im Wesentlichen zueinander in Flucht bzw. auf einer Geraden.

Die Vorbereitungs- und Positionierungsvorrichtung 14 weist eine formstabile Unterlage 22 auf, die für die Aufnahme, Vorbereitung und Positionierung eines Patienten 12 in dem Vorbereitungsbereich 16 vorgesehen ist.

Darüber hinaus umfasst die Vorbereitungs- und Positionierungsvorrichtung 14 in erfindungswesentlicher Weise eine linear geführte Teleskopeinrichtung 24, welche die formstabile Unterlage 22 abstützt. Die linear geführte Teleskopeinrichtung 24 dient dazu, den Patienten 12, der in dem Vorbereitungsbereich 16 auf der formstabilen Unterlage 22 vorbereitet und positioniert wurde, aus dem Vorbereitungsbereich 16 zu der Behandlungs- und/oder Versorgungsvorrichtung 18 in dem Behandlungsbereich 20, und umgekehrt, zu verbringen bzw. zu transportieren.

Die linear geführte Teleskopeinrichtung 24 ist bei der in den Fig. 1A bis 1H dargestellten beispielhaften Ausführungsform der Anordnung 10 teilweise auf einer Basis 26 oder einem Fundament des Vorbereitungsbereiches 16 bzw. Vorbereitungsraumes abgestützt und führbar.

Eine solche teilweise Abstützung der Teleskopeinrichtung 26 kommt vorzugsweise zur Anwendung, sofern die auftretenden Kräfte und Momente aufgrund der Entfernungs- und Raumverhältnisse von und/oder zwischen dem Vorbereitungsbereich 16 und dem Behandlungsbereich 20 nicht übermäßig groß sind. Die teilweise Abstützung der Teleskopeinrichtung 26 hat den Vorteil, eine absolut erschütterungsfreie Verbringung des Patienten 12 aus dem Vorbereitungsbereich 16 in den Behandlungsbereich 20, und umgekehrt, sicherzustellen, ohne eine hohe Maß- und Toleranzgenauigkeit zwischen der Teleskopeinrichtung 24 und der Basis 24 vorzusehen. Ohne im Einzelnen dargestellt zu sein, ist es jedoch in konstruktiver Abwandlung dazu ebenso möglich, die linear geführte Teleskopeinrichtung 24 vollständig auf der Basis 26 oder dem Fundament abzustützen, zu führen und zu bewegen.

Die Vorbereitungs- und Positionierungsvorrichtung 14 ist vorzugsweise derart ausgebildet, dass die formstabile Unterlage 22, die von der linear geführten Teleskopeinrichtung 24 abgestützt wird, erschütterungsfrei und/oder vollautomatisch aus dem Vorbereitungsbereich 16 in den Behandlungsbereich 20, und umgekehrt, verbringbar ist.

Ohne im Einzelnen gezeigt oder im Detail beschrieben zu sein, ist die linear geführte Teleskopeinrichtung 24 elektromotorisch und/oder pneumatisch und/oder hydraulisch antreibbar ausgebildet.

Entsprechend den Fig. 1A und 1B befindet sich die beispielhafte Anordnung 10 in der Anfangsstellung. Die linear geführte Teleskopeinrichtung 24 ist vollends zurückgezogen bzw. eingefahren. Auf der formstabilen Unterlage 22 in dem Vorbereitungsbereich 16 wird der Patient 12 vorbereitet und positioniert.

In den Fig. 1C und 1D befindet sich die beispielhafte Anordnung 10 in einer Zwischenstellung. Die linear geführte Teleskopeinrichtung 24 ist teilweise, wie hier dargestellt nahezu vollständig, ausgefahren. Die Unterlage 22 wird gerade aus dem Vorbereitungsbereich 16 in den Behandlungsbereich 20 verbracht bzw. transportiert.

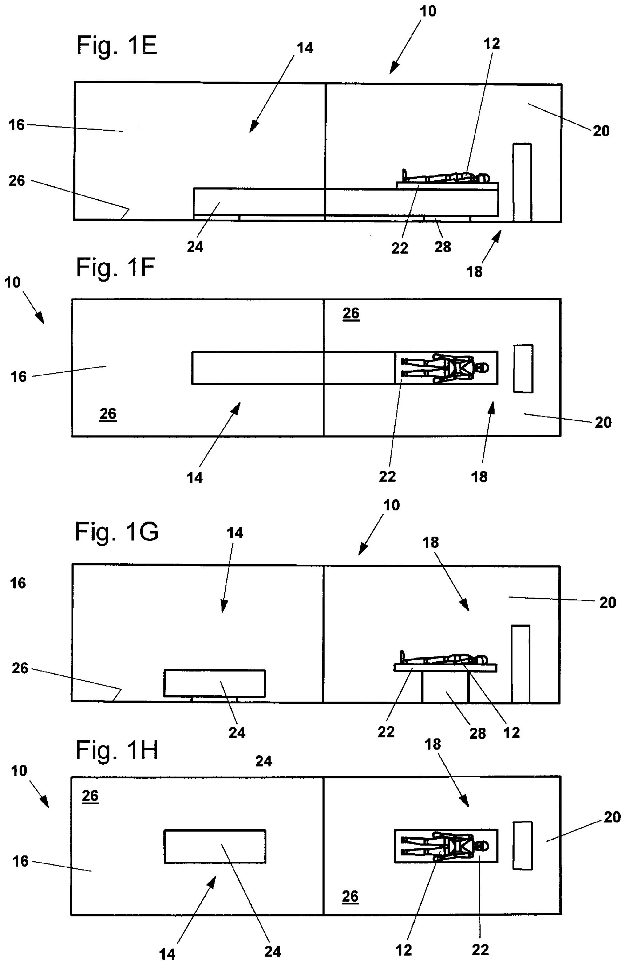

Wie die Fig. 1E und 1F zeigen, befindet sich die beispielhafte Anordnung 10 in einer Endstellung oder in einer weiteren Zwischenstellung. Dabei ist die linear geführte Teleskopeinrichtung 24 vollständig ausgefahren. Die Unterlage 22 ist vollends in den Behandlungsbereich 20 verbracht bzw. transportiert und der Behandlungs- und/oder Versorgungsvorrichtung 18 unmittelbar zugeordnet. Der Patient 12 auf der Unterlage 22 steht für den medizinischen Eingriff bzw. die medizinische Versorgung bereit.

Die formstabile Unterlage 22 und die linear geführte Teleskopeinrichtung 24 sind zweckmäßigerweise miteinander unlösbar verbunden. Entsprechend den Fig. 1E und 1F hat die linear geführte Teleskopeinrichtung 24 ihre Endstellung erreicht und ist vollständig ausgefahren.

Demgegenüber können die formstabile Unterlage 22 und die linear geführte Teleskopeinrichtung 24 jedoch auch miteinander lösbar verbunden sein. Gemäß den Fig. 1G und 1H ist es auf diese Weise möglich, die formstabile Unterlage 22 von der linear geführten Teleskopeinrichtung 24 auf einen Unter- oder Gestelltisch 28 der Behandlungs- und/oder Versorgungsvorrichtung 18 abzusetzen. Die formstabile Unterlage 22 wird von dem Untertisch 28 lösbar aufgenommen und abgestützt. Entsprechend den Fig. 1G und 1H hat die linear geführte Teleskopeinrichtung 24 insoweit lediglich eine andere Zwischenstellung eingenommen und verfährt sodann aus dem Behandlungsbereich 20 in den Vorbereitungsbereich 16 zurück, um ihre weitere, hier eigentliche, Endstellung einzunehmen.

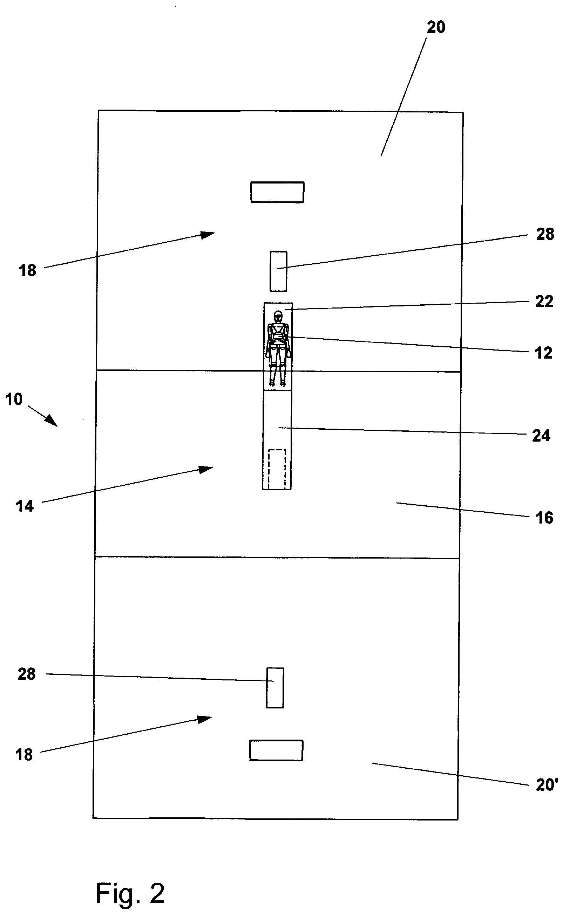

In der Fig. 2 ist eine weitere beispielhafte Ausführungsform der Anordnung 10 beispielhaft dargestellt.

Die beispielhafte Ausführungsform der Anordnung 10 nach der Fig. 2 umfasst wiederum eine Vorbereitungs- und Positionierungsvorrichtung 14 und zwei Behandlungs- und/oder Versorgungsvorrichtungen 18, 18'. Mithin weist die Anordnung 10 eine weitere - sprich zweite - Behandlungs- und/oder Versorgungsvorrichtung 18' auf.

Die Vorbereitungs- und Positionierungsvorrichtung 14 ist dabei zwischen den zwei Behandlungs- und/oder Versorgungsvorrichtungen 18, 18' angeordnet. Die erfindungsgemäße Anordnung 10 der Fig. 2 befindet sich in einer Zwischenstellung. Die linear geführte Teleskopeinrichtung 24 ist teilweise, d.h. annähernd vollständig, ausgefahren. Die Unterlage 22 wird gerade aus dem Vorbereitungsbereich 16 in den Behandlungsbereich 20 verbracht bzw. transportiert.

Auf diese Weise ergibt sich die Möglichkeit, die formstabile Unterlage 22 von der linear geführten Teleskopeinrichtung 24 auf den Unter- oder Gestelltisch 28 der Behandlungs- und/oder Versorgungsvorrichtung 18 abzusetzen und die linear geführte Teleskopeinrichtung 24 insoweit anschließend aus dem Behandlungsbereich 20 in den Vorbereitungsbereich 18 zurückzuverfahren. Während der medizinischen Behandlung und/oder Versorgung dieses Patienten 12 kann sodann in dem Vorbereitungsbereich 16 ein anderer Patient 12 (nicht dargestellt) auf der formstabilen Unterlage 22 vorbereitet und positioniert werden. Dieser andere Patient 12 wird schließlich der Behandlungs- und/oder Versorgungsvorrichtung 18' mit der formstabilen Unterlage 22 von der linear geführten Teleskopeinrichtung 24 zugeführt, um die medizinische Behandlung und/oder Versorgung an diesem anderen Patienten 12, d.h. zeitgleich zu der Behandlung und/oder Versorgung des Patienten 12 mittels Behandlungs- und/oder Versorgungsvorrichtung 18, durchzuführen.

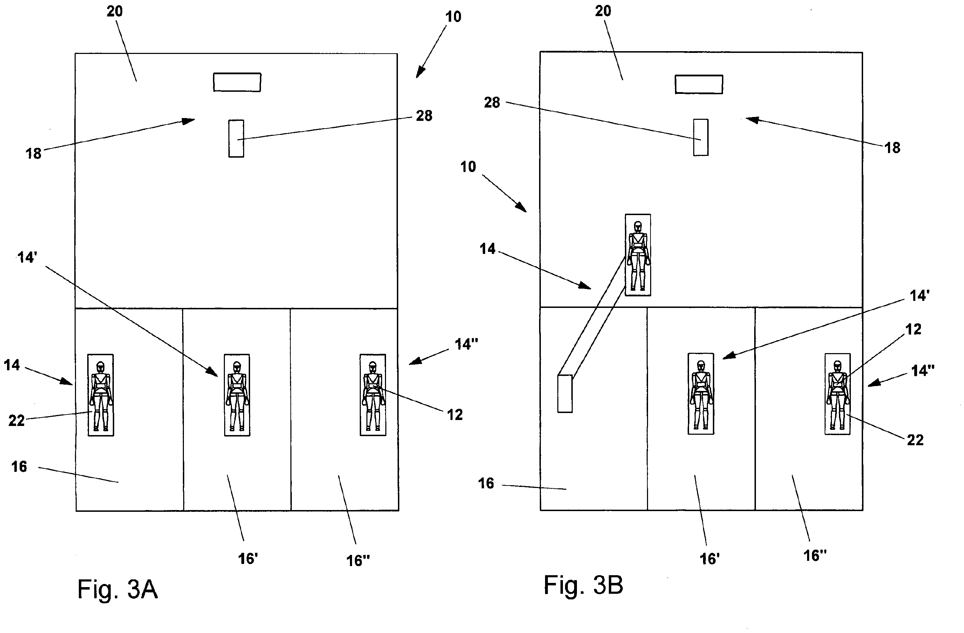

Aus den Fig. 3A und 3B geht eine Ausführungsform der erfindungsgemäßen Anordnung 10 hervor.

Die Ausführungsform der Anordnung 10 nach den Fig. 3A und 3B ist mit insgesamt drei Vorbereitungs- und Positionierungsvorrichtungen 14, 14', 14" und einer Behandlungs- und/oder Versorgungsvorrichtung 18 ausgestattet.

Die drei Vorbereitungs- und Positionierungsvorrichtungen 14, 14', 14" sind parallel zueinander angeordnet. Die jeweiligen Patienten 12 können somit auf den formstabilen Unterlagen 22 der entsprechenden Vorbereitungs- und Positionierungsvorrichtung 14, 14', 14" vorbereitet und positioniert werden, und zwar zeitgleich, nahezu zeitgleich oder in zueinander genau abgestimmter, zeitlicher Aufeinanderfolge.

Wie die Fig. 3B zeigt, befindet sich die erfindungsgemäße Anordnung 10 in einer Zwischenstellung. Die linear geführte Teleskopeinrichtung 24 einer, nämlich der linken bzw. obersten, der drei Vorbereitungs- und Positionierungsvorrichtungen 14, 14', 14" ist teilweise ausgefahren. Die Unterlage 22 wird gerade aus dem Vorbereitungsbereich 16 in den Behandlungsbereich 20 verbracht bzw. transportiert.

Zusätzlich sind die formstabile Unterlage 22 und die linear geführte Teleskopeinrichtung 24 vorzugsweise, ohne im Einzelnen dargestellt zu sein, zueinander relativ verdrehbar ausgebildet. Hierdurch lässt sich - wenn gewünscht - zum Beispiel eine translatorische Bewegung der formstabilen Unterlage 22 erreichen. Die Bewegung der Unterlage 22 ist vergleichmäßigt und zugleich definiert, um eine gewünschte Relativstellung zwischen der formstabilen Unterlage 22 und der linear geführten Teleskopeinrichtung 24 in dem Vorbereitungsbereich 16 einerseits sowie der formstabilen Unterlage 22 und der zugeordneten Behandlungs- und/oder Versorgungsvorrichtung 18 bzw. deren Unter- oder Gestelltisch 28 in dem Behandlungsbereich 20 andererseits zu erhalten.

In alternativer oder kumulativer Ausgestaltung dazu kann die formstabile Unterlage 22 und der Unter- oder Gestelltisch 28 zueinander relativ verdrehbar ausgebildet sein. Ebenso denkbar ist es, alternativ oder kumulativ den Unter- oder Gestelltisch 28 der Behandlungs- und/oder Versorgungsvorrichtung 18 und ein/e den Unter- oder Gestelltisch 28 abstützende/s Basis 26 oder Fundament der mindestens einen Behandlungs- und/oder Versorgungsvorrichtung 18 relativ verdrehbar auszubilden.

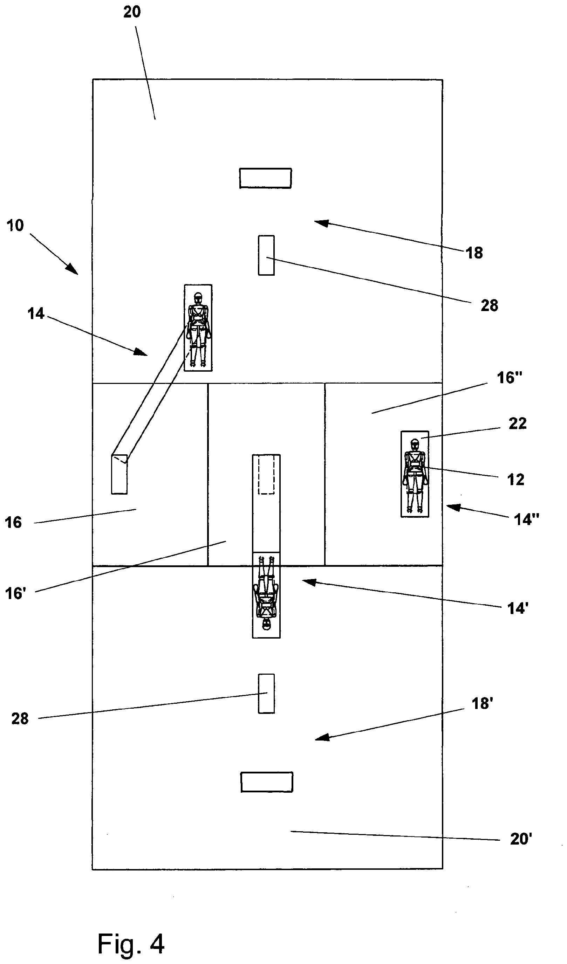

In der Fig. 4 ist eine andere Ausführungsform der erfindungsgemäßen Anordnung 10 gezeigt, welche eine Kombination der beiden Ausführungsformen der Fig. 2 und 3A sowie 3B darstellt.

Demnach sind durch die Ausführungsform der Anordnung 10 der Fig. 4 insgesamt drei Vorbereitungs- und Positionierungsvorrichtungen 14, 14', 14" und zwei Behandlungs- und/oder Versorgungsvorrichtungen 18, 18' vorgeschlagen.

Die drei Vorbereitungs- und Positionierungsvorrichtungen 14, 14', 14" sind zueinander parallel und zwischen den zwei Behandlungs- und/oder Versorgungsvorrichtungen 18, 18' angeordnet. Wie der Fig. 4 entnehmbar ist, befindet sich die erfindungsgemäße Anordnung 10 der Fig. 4 in einer Zwischenstellung.

Zwei linear geführte Teleskopeinrichtungen 24 sind teilweise, d.h. annähernd vollständig, ausgefahren. Die Unterlagen 22 werden gerade aus dem Vorbereitungsbereich 16 bzw. 16' in den Behandlungsbereich 20 bzw. 20' verbracht bzw. transportiert.

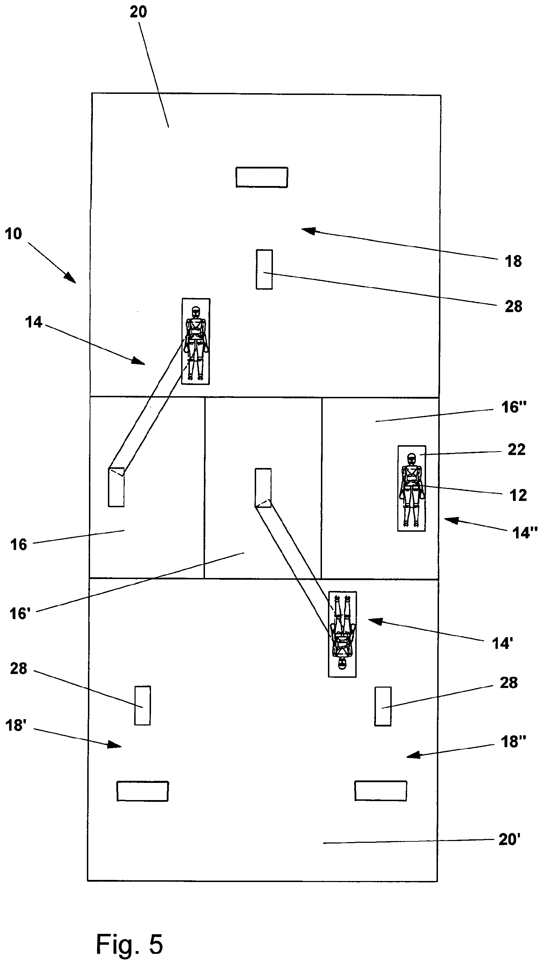

In den Fig. 5 bis 7 sind ist noch andere Ausführungsformen der erfindungsgemäßen Anordnung 10 in Abänderung von Derjenigen nach der Fig. 4 dargestellt.

Im Unterschied zu der Ausführungsform der Anordnung 10 nach der Fig. 4 sind in dem Behandlungsbereich 20' bei der Ausführung der Anordnung 10 der Fig. 5 zwei Behandlungs- und/oder Versorgungsvorrichtungen 18', 18" angeordnet, die zueinander parallel oder im Wesentlichen parallel ausgerichtet sind.

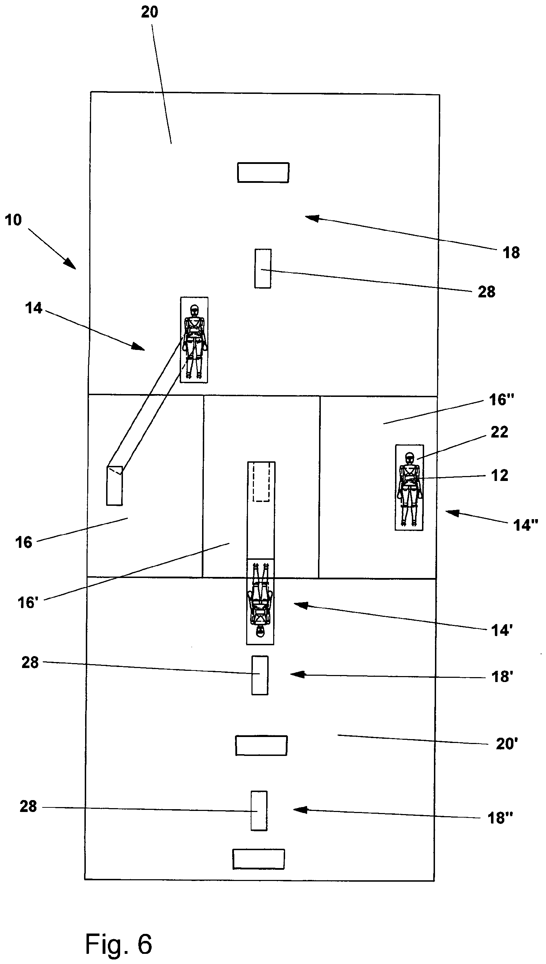

Die Ausführung der Anordnung 10 der Fig. 6 unterscheidet sich von der Ausführungsform der Anordnung 10 nach der Fig. 4 dadurch, dass in dem Behandlungsbereich 20' zwei Behandlungs- und/oder Versorgungsvorrichtungen 18', 18" hintereinander oder im Wesentlichen hintereinander angeordnet sind.

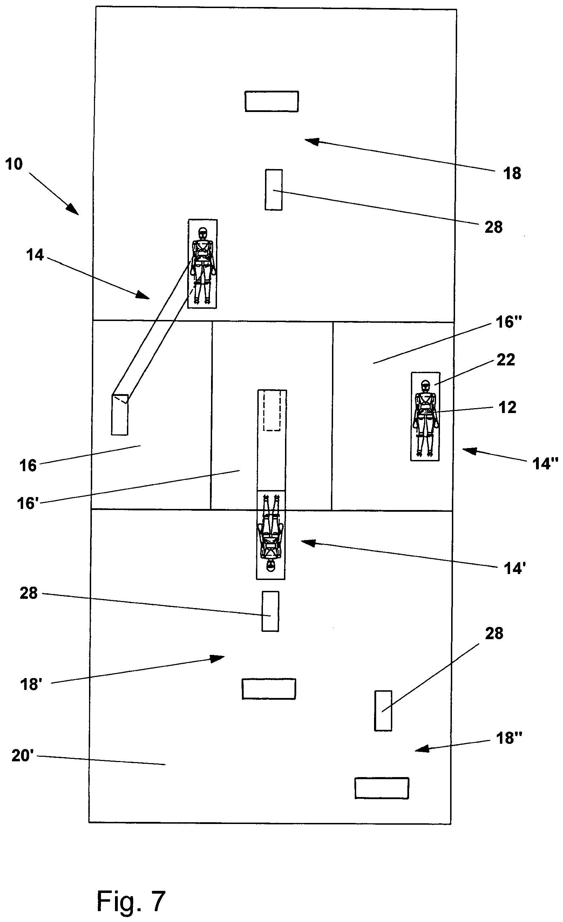

Im Ausführungsbeispiel der Anordnung 10, das in der Fig. 7 ersichtlich ist, sind in dem Behandlungsbereich 20' zwei Behandlungs- und/oder Versorgungsvorrichtungen 18, 18' angeordnet, die zueinander versetzt sind.

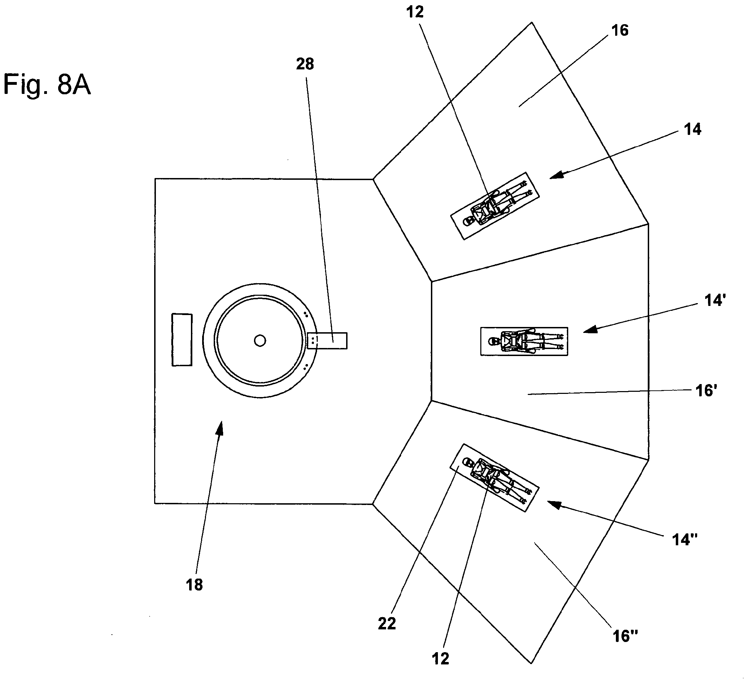

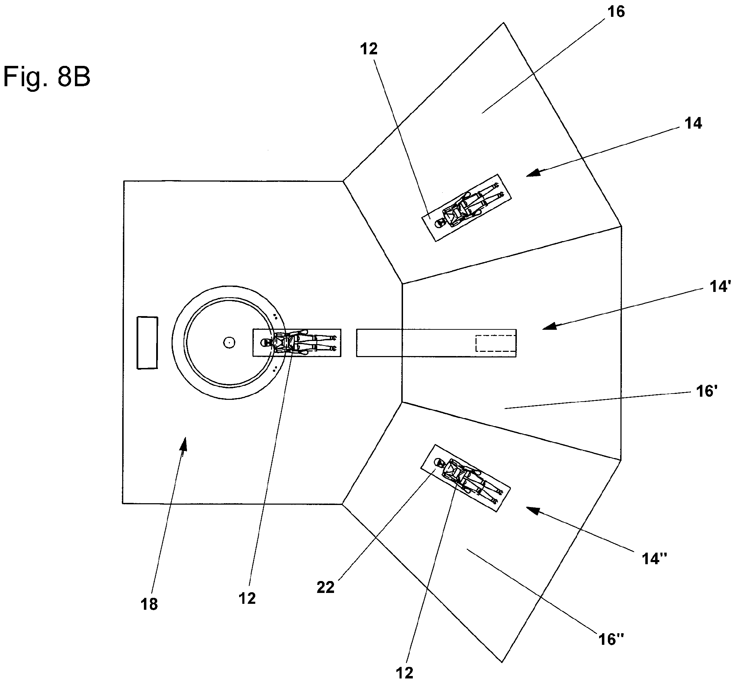

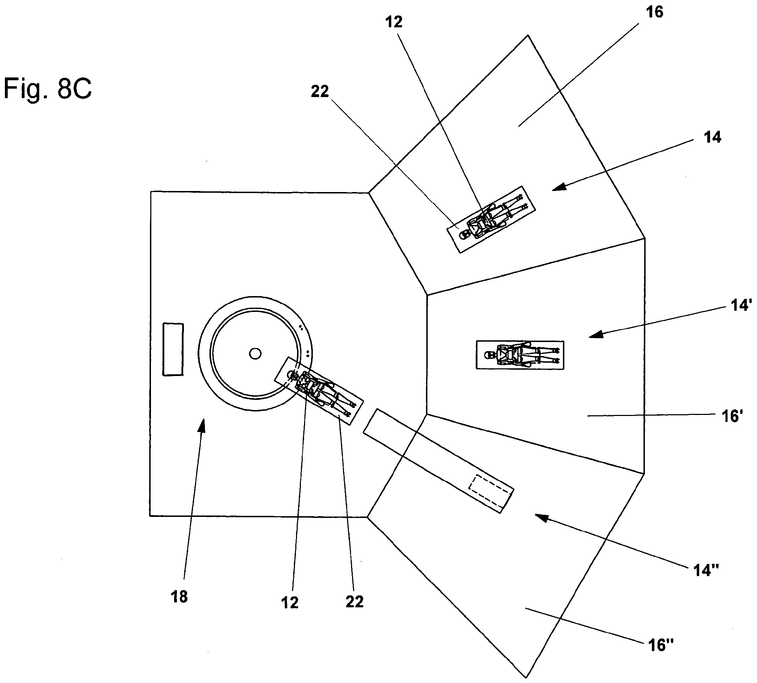

Schließlich zeigen die Fig. 8A, 8B und 8C eine noch andere Ausführungsform einer erfindungsgemäßen Anordnung 10.

Bei der Ausführung nach den Fig. 8A bis 8C sind demnach zwei oder mehrere, hier insgesamt drei, Vorbereitungs- und Positionierungsvorrichtungen 14, 14', 14" im Wesentlichen auf einem Teilkreis nebeneinander um eine Behandlungs- und/oder Versorgungsvorrichtung 18 angeordnet. Wie den Fig. 8B und 8C entnehmbar ist, befindet sich die erfindungsgemäße Anordnung 10 den Fig. 8B und 8C jeweils in einer Zwischenstellung. Zwei linear geführte Teleskopeinrichtungen 24 werden gerade in die Endstellung zurückgezogen bzw. zurückgefahren, nachdem die formstabile Unterlage 24 mit dem Patienten 12 auf dem Unter- oder Gestelltisch 28 der Behandlungs- und/oder Versorgungsvorrichtung 18 abgesetzt wurde. Der Unter- oder Gestelltisch 28 ist zwischen den beiden Stellungen nach den Fig. 8B und 8C relativ verdreht.

Die vorliegende Erfindung ist nicht auf die dargestellten Ausführungsformen der Anordnung 10 beschränkt. So ist es ohne weiteres möglich, die Anordnung 10 abweichend von den vorstehend beschriebenen Ausführungsformen nach den Fig. 8A bis 8C zwei oder mehrere Vorbereitungs- und Positionierungsvorrichtungen 14, 14', 14" im Wesentlichen in einem Kreis nebeneinander, also kreisförmig, um die mindestens eine Behandlungs- und/oder Versorgungsvorrichtung 18 anzuordnen. Ohne im Einzelnen dargestellt zu sein, können die Vorbereitungs- und Positionierungsvorrichtungen 14, 14', 14" zueinander identisch ausgebildet sein. Die Behandlungs- und/oder Versorgungsvorrichtungen 18, 18', 18" können zueinander identisch oder unterschiedlich ausgebildet sein.

## Patentansprüche

1. Anordnung zum Vorbereiten und Positionieren von medizinisch zu behandelnden und/oder zu versorgenden Patienten (12) umfassend wenigstens zwei Vorbereitungsbereiche (16, 16', 16") mit mindestens einer in dem jeweiligen Vorbereitungsbereich (16, 16', 16") angeordneten Vorrichtung (14, 14', 14") zum Vorbereiten und Positionieren eines Patienten (12) und wenigstens einen Behandlungsbereich (20, 20') mit mindestens einer in dem jeweiligen Behandlungsbereich (20, 20') angeordneten Vorrichtung (18, 18', 18") zur medizinischen Behandlung und/oder Versorgung des Patienten (12), wobei die Vorbereitungs- und Behandlungsbereiche (16, 16', 16", 20, 20') voneinander in ihrer Funktion unterschiedlich und räumlich getrennt sowie zur Vermeidung einer gegenseitigen Einsichtmöglichkeit durch andere Patienten durch mobile oder feste Wände voneinander abgeteilt sind, wobei die wenigstens zwei Vorbereitungs- und Positionierungsvorrichtungen (14, 14', 14") eine formstabile Unterlage (22) zum Aufnehmen, Vorbereiten und Positionieren des Patienten (12) sowie dessen Ausrichten entsprechend der vorzusehenden Behandlungs- und/oder Versorgungsmaßnahmen in dem Vorbereitungsbereich (16, 16', 16") umfassen, **dadurch gekennzeichnet, dass** die wenigstens zwei Vorbereitungs- und Positionierungsvorrichtungen (14, 14', 14") eine die formstabile Unterlage (22) abstützende, linear geführte Teleskopeinrichtung (24) umfassen, durch welche die formstabile Unterlage (22) mit dem in dem jeweiligen Vorbereitungsbereich (16, 16', 16") auf der formstabilen Unterlage (22) vorbereiteten und positionierten Patienten (12) ohne Bodenführung und somit frei von möglicherweise durch Bodenunebenheiten hervorgerufenen Erschütterungen aus dem jeweiligen Vorbereitungsbereich (16, 16', 16") zu der mindestens einen Behandlungs- und/oder Versorgungsvorrichtung (18, 18', 18") in dem Behandlungsbereich (20, 20'), und umgekehrt, verbringbar ist.

2. Anordnung nach Anspruch 1, **dadurch gekennzeichnet, dass** die wenigstens zwei Vorbereitungs- und Positionierungsvorrichtungen (14, 14', 14") und die mindestens eine Behandlungs- und/oder Versorgungsvorrichtung (18, 18', 18") zueinander linear angeordnet sind.

3. Anordnung nach Anspruch 1 oder 2, **dadurch gekennzeichnet, dass** die wenigstens zwei Vorbereitungs- und Positionierungsvorrichtungen (14, 14', 14") zwischen zwei oder mehreren Behandlungs- und/oder Versorgungsvorrichtungen (18, 18', 18") angeordnet ist.

4. Anordnung nach einem der Ansprüche 1 bis 3, **dadurch gekennzeichnet, dass** zwei oder mehrere Vorbereitungs- und Positionierungsvorrichtungen (14, 14', 14") parallel zueinander angeordnet sind.

5. Anordnung nach einem der Ansprüche 1 bis 4, **dadurch gekennzeichnet, dass** zwei oder mehrere Vorbereitungs- und Positionierungsvorrichtungen (14, 14', 14") im Wesentlichen auf einem Teilkreis oder in einem Kreis nebeneinander um die mindestens eine Behandlungs- und/oder Versorgungsvorrichtung (18, 18', 18") angeordnet sind.

6. Anordnung nach einem der Ansprüche 1 bis 5, **dadurch gekennzeichnet, dass** zwei oder mehrere Behandlungs- und/oder Versorgungsvorrichtungen (18, 18', 18") parallel nebeneinander und/oder hintereinander und/oder versetzt zueinander angeordnet sind.

7. Anordnung nach einem der Ansprüche 1 bis 6, **dadurch gekennzeichnet, dass** die wenigstens zwei Vorbereitungs- und Positionierungsvorrichtungen (14, 14', 14") derart ausgebildet sind, dass die von der linear geführten Teleskopeinrichtung (24) abgestützte formstabile Unterlage (22) der wenigstens zwei Vorbereitungs- und Positionierungsvorrichtungen (14, 14', 14") vollautomatisch aus dem Vorbereitungsbereich (16, 16', 16") in den Behandlungsbereich (20, 20'), und umgekehrt, verbringbar ist.

8. Anordnung nach einem der Ansprüche 1 bis 7, **dadurch gekennzeichnet, dass** die linear geführte Teleskopeinrichtung (24) der wenigstens zwei Vorbereitungs- und Positionierungsvorrichtungen (14, 14', 14") elektromotorisch und/oder pneumatisch und/oder hydraulisch antreibbar ist.

9. Anordnung nach einem der Ansprüche 1 bis 8, **dadurch gekennzeichnet, dass** die linear geführte Teleskopeinrichtung (24) der wenigstens zwei Vorbereitungs- und Positionierungsvorrichtungen (14, 14', 14") vollständig oder teilweise auf einer Basis (26) oder einem Fundament abstützbar und führbar ist.

10. Anordnung nach einem der Ansprüche 1 bis 9, **dadurch gekennzeichnet, dass** die formstabile Unterlage (22) und die linear geführte Teleskopeinrichtung (24) der wenigstens zwei Vorbereitungs- und Positionierungsvorrichtungen (14, 14', 14") miteinander unlösbar verbunden sind.

11. Anordnung nach einem der Ansprüche 1 bis 9, **dadurch gekennzeichnet, dass** die formstabile Unterlage (22) und die linear geführte Teleskopeinrichtung (24) der wenigstens zwei Vorbereitungs- und Positionierungsvorrichtungen (14, 14', 14") miteinander lösbar verbunden sind und die formstabile Unterlage (22) von der linear geführten Teleskopeinrichtung (24) auf einen Unter- oder Gestelltisch (28) der mindestens einen Behandlungs- und/oder Versorgungsvorrichtung (18, 18', 18") absetzbar und von dem Unter- oder Gestelltisch (28) lösbar aufnehmbar und abstützbar ist.

12. Anordnung nach einem der Ansprüche 1 bis 11, **dadurch gekennzeichnet, dass** die formstabile Unterlage (22) und die linear geführte Teleskopeinrichtung (24) der wenigstens zwei Vorbereitungs- und Positionierungsvorrichtungen (14, 14', 14") zueinander relativ verdrehbar ausgebildet sind.

13. Anordnung nach einem der Ansprüche 1 bis 12, **dadurch gekennzeichnet, dass** die formstabile Unterlage (22) der wenigstens zwei Vorbereitungs- und Positionierungsvorrichtungen (14, 14', 14") und der Unter- oder Gestelltisch (28) der mindestens einen Behandlungs- und/oder Versorgungsvorrichtung (18, 18', 18") zueinander relativ verdrehbar ausgebildet sind und/oder der Unter- oder Gestelltisch (28) der mindestens einen Behandlungs- und/oder Versorgungsvorrichtung (18, 18', 18") und ein/e den Unter- oder Gestelltisch (28) abstützende/s Basis (26) oder Fundament der mindestens einen Behandlungs- und/oder Versorgungsvorrichtung (18, 18', 18") relativ verdrehbar ausgebildet sind.

14. Anordnung nach einem der Ansprüche 1 bis 13, **dadurch gekennzeichnet, dass** die Vorbereitungs- und Positionierungsvorrichtungen (14, 14', 14") zueinander identisch ausgebildet sind und/oder dass die Behandlungs- und/oder Versorgungsvorrichtungen (18, 18', 18") zueinander identisch oder unterschiedlich ausgebildet sind.

## Claims

1. Arrangement for preparing and positioning patients (12) to be treated and/or cared for medically, comprising at least two preparation areas (16, 16', 16") with at least one device (14, 14', 14") arranged in the respective preparation area (16, 16', 16") for preparing and positioning of a patient (12) and at least one treatment area (20, 20') with at least one device (18, 18', 18") arranged in the respective treatment area (20, 20') for medical treatment and/or care of the patient (12), with the preparation and treatment areas (16, 16', 16", 20, 20') differing from each other in their functions and being spatially separated, as well as partitioned from each other by movable or fixed walls to prevent mutual views by other patients, wherein the at least two preparation and positioning devices (14, 14', 14") comprise a dimensionally stable base (22) for receiving, preparing and positioning the patient (12) as well as his orientation in the preparation area (16, 16', 16") according to the intended treatment and/or care measures, **characterized in that** the at least two preparation and positioning devices (14, 14', 14") comprise a telescopic device (24) with linear guide that supports the dimensionally stable base (22), through which the dimensionally stable base (22) with the patient (12) prepared and positioned on the dimensionally stable base (22) in the respective preparation area (16, 16', 16") can be moved from the respective preparation area (16, 16', 16") to the at least one treatment and/or care device (18, 18', 18") in the treatment area (20, 20'), and vice versa, without floor guide, and thus free of any shocks that may be caused by irregularities of the floor.

2. Arrangement according to claim 1, **characterized in that** the at least two preparation and positioning devices (14, 14', 14") and the at least one treatment and/or care device (18, 18', 18") are in linear position to each other.

3. Arrangement according to claim 1 or 2, **characterized in that** the at least two preparation and positioning devices (14, 14', 14") are arranged between two or more treatment and/or care devices (18, 18', 18").

4. Arrangement according to one of claims 1 to 3, **characterized in that** two or more preparation and positioning devices (14, 14', 14") are arranged parallel to each other.

5. Arrangement according to one of claims 1 to 4, **characterized in that** two or more preparation and positioning devices (14, 14', 14") essentially are arranged in a semicircle or in a circle next to each other around the at least one treatment and/or care device (18, 18', 18").

6. Arrangement according to one of claims 1 to 5, **characterized in that** two or more treatment and/or care devices (18, 18', 18") are arranged parallel to each other and/or one behind the other and/or in staggered arrangement.

7. Arrangement according to one of claims 1 to 6, **characterized in that** the at least two preparation and positioning devices (14, 14', 14") are designed in such manner that the dimensionally stable base (22) supported by the linearly guided telescopic device (24) of the at least two preparation and positioning devices (14, 14', 14") is fully automatically moveable from the preparation area (16, 16', 16") to the treatment area (20, 20'), and vice versa.

8. Arrangement according to one of claims 1 to 7, **characterized in that** the linearly guided telescopic device (24) of the at least two preparation and positioning devices (14, 14', 14") is actuatable by electric motor and/or pneumatics and/or hydraulics.

9. Arrangement according to one of claims 1 to 8, **characterized in that** the linearly guided telescopic device (24) of the at least two preparation and positioning devices (14, 14', 14") is entirely or partially supportable and guidable on a basis (26) or foundation.

10. Arrangement according to one of claims 1 to 9, **characterized in that** the dimensionally stable base (22) and the linearly guided telescopic device (24) of the at least two preparation and positioning devices (14, 14', 14") are inseparably coupled to each other.

11. Arrangement according to one of claims 1 to 9, **characterized in that** the dimensionally stable base (22) and the linearly guided telescopic device (24) of the at least two preparation and positioning devices (14, 14', 14") are separably connected with each other and the dimensionally stable base (22) is placeable by the linearly guided telescopic device (24) on a supporting or bearing table (28) of the at least one treatment and/or care device (18, 18', 18") and is separably receivable and supportable by the supporting or bearing table (28).

12. Arrangement according to one of claims 1 to 11, **characterized in that** the dimensionally stable base (22) and the linearly guided telescopic device (24) of the at least two preparation and positioning devices (14, 14', 14") are designed relatively rotatable to each other.

13. Arrangement according to one of claims 1 to 12, **characterized in that** the dimensionally stable base (22) of the at least two preparation and positioning devices (14, 14', 14") and the supporting or bearing table (28) of the at least one treatment and/or care device (18, 18', 18") are designed relatively rotatable to each other and/or the supporting or bearing table (28) of the at least one treatment and/or care device (18, 18', 18") and a basis (26) or foundation supporting the supporting or bearing table (28) of the at least one treatment and/or care device (18, 18', 18") are designed relatively rotatable to each other.

14. Arrangement according to one of claims 1 to 13, **characterized in that** the preparation and positioning devices (14, 14', 14") are designed identically to each other and/or that the treatment and/or care devices (18, 18', 18") are designed identically or differently to each other.

## Revendications

1. Installation de préparation et de positionnement de patients (12) à traiter et/ou à soigner médicalement, comportant au moins deux zones de préparation (16, 16', 16") pourvues d'au moins un dispositif (14, 14', 14") agencé dans la zone de préparation respective (16, 16', 16") et destiné à préparer et à positionner un patient (12) et au moins une zone de traitement (20, 20') pourvues d'au moins un dispositif (18, 18', 18") agencé dans la zone de traitement respective (20, 20') et destiné au traitement et/ou aux soins médicaux du patient (12), les zones de préparation et de traitement (16, 16', 16", 20, 20') étant différentes quant à leur fonction et étant séparées dans l'espace et subdivisées les unes des autres par des parois mobiles ou fixes afin d'éviter une possibilité de vision mutuelle par d'autres patients, lesdits au moins deux dispositifs de préparation et de positionnement (14, 14', 14") comprenant un support (22) de forme stable pour recevoir, préparer et positionner le patient (12) et pour l'orienter selon les mesures de traitement et/ou les soins à prévoir dans la zone de préparation (16, 16', 16"), **caractérisée en ce que** lesdits au moins deux dispositifs de préparation et de positionnement (14, 14', 14") comprennent un moyen télescopique (24) soutenant le support (22) de forme stable et guidé linéairement permettant d'amener le support (22) de forme stable avec le patient (12) préparé et positionné dans la zone de préparation respective (16, 16', 16") sur le support (22) de forme stable sans guidage sur le sol et donc sans secousses éventuelles causées par des irrégularités du sol, depuis la zone de préparation respective (16, 16', 16'') jusqu'audit au moins un dispositif de traitement et/ou de soins (18, 18', 18") dans la zone de traitement (20, 20'), et inversement.

2. Installation selon la revendication 1, **caractérisée en ce que** lesdits au moins deux dispositifs de préparation et de positionnement (14, 14', 14") et ledit au moins un dispositif de traitement et/ou de soins (18, 18', 18") sont agencés linéairement les uns par rapport aux autres.

3. Installation selon la revendication 1 ou 2, **caractérisée en ce que** lesdits au moins deux dispositifs de préparation et de positionnement (14, 14', 14") sont agencés entre deux ou plusieurs dispositifs de traitement et/ou de soins (18, 18', 18").

4. Installation selon l'une des revendications 1 à 3, **caractérisée en ce que** deux ou plusieurs dispositifs de préparation et de positionnement (14, 14', 14") sont agencés parallèlement les uns par rapport aux autres.

5. Installation selon l'une des revendications 1 à 4, **caractérisée en ce que** deux ou plusieurs dispositifs de préparation et de positionnement (14, 14', 14") sont agencés sensiblement sur un cercle partiel ou dans un cercle les uns à côté des autres autour dudit au moins un dispositif de traitement et/ou de soins (18, 18', 18").

6. Installation selon l'une des revendications 1 à 5, **caractérisée en ce que** deux ou plusieurs dispositifs de traitement et/ou de soins (18, 18', 18") sont agencés parallèlement les uns à côté des autres et/ou les uns derrière les autres et/ou en décalage les uns par rapport aux autres.

7. Installation selon l'une des revendications 1 à 6, **caractérisée en ce que** lesdits au moins deux dispositifs de préparation et de positionnement (14, 14', 14") sont réalisés de telle sorte que le support (22) de forme stable desdits au moins deux dispositifs de préparation et de positionnement (14, 14', 14"), support qui est soutenu par le moyen télescopique (24) guidé linéairement, est susceptible d'être amené de manière complètement automatique depuis la zone de préparation (16, 16', 16") jusque dans la zone de traitement (20, 20'), ou inversement.

8. Installation selon l'une des revendications 1 à 7, **caractérisée en ce que** le moyen télescopique (24) guidé linéairement desdits au moins deux dispositifs de préparation et de positionnement (14, 14', 14") est susceptible d'être entraîné par moteur électrique et/ou par voie pneumatique et/ou par voie hydraulique.

9. Installation selon l'une des revendications 1 à 8, **caractérisée en ce que** le moyen télescopique (24) guidé linéairement desdits au moins deux dispositifs de préparation et de positionnement (14, 14', 14") est susceptible d'être soutenu et guidé complètement ou partiellement sur une base (26) ou sur une fondation.

10. Installation selon l'une des revendications 1 à 9, **caractérisée en ce que** le support (22) de forme stable et le moyen télescopique (24) guidé linéairement desdits au moins deux dispositifs de préparation et de positionnement (14, 14', 14") sont reliés entre eux de manière non détachable.

11. Installation selon l'une des revendications 1 à 9, **caractérisée en ce que** le support (22) de forme stable et le moyen télescopique (24) guidé linéairement desdits au moins deux dispositifs de préparation et de positionnement (14, 14', 14") sont reliés entre eux de façon détachable et le support (22) de forme stable est susceptible d'être posé depuis le moyen télescopique (24) guidé linéairement sur une table de base ou de bâti (28) dudit au moins un dispositif de traitement et/ou de soins (18, 18', 18") et d'être reçu et soutenu de façon détachable par la table de base ou de bâti (28).

12. Installation selon l'une des revendications 1 à 11, **caractérisée en ce que** le support (22) de forme stable et le moyen télescopique (24) guidé linéairement desdits au moins deux dispositifs de préparation et de positionnement (14, 14', 14") sont réalisés de façon mobile en rotation l'un par rapport à l'autre.

13. Installation selon l'une des revendications 1 à 12, **caractérisée en ce que** le support (22) de forme stable desdits au moins deux dispositifs de préparation et de positionnement (14, 14', 14") et la table de base ou de bâti (28) dudit au moins un dispositif de traitement et/ou de soins (18, 18', 18") sont réalisés de façon mobile en rotation l'un par rapport à l'autre et/ou la table de base ou de bâti (28) dudit au moins un dispositif de traitement et/ou de soins (18, 18', 18") et une base (26) ou une fondation, soutenant la table de base ou de bâti (28), dudit au moins un dispositif de traitement et/ou de soins (18, 18', 18") sont réalisés de façon mobile en rotation l'une par rapport à l'autre.

14. Installation selon l'une des revendications 1 à 13, **caractérisée en ce que** les dispositifs de préparation et de positionnement (14, 14', 14") sont réalisés de façon identique entre eux et/ou **en ce que** les dispositifs de traitement et/ou de soins (18, 18', 18") sont réalisés de façon identique ou différente entre eux.
